# EUROPEAN PATENT APPLICATION

(11) **EP 2 641 597 A1**
(43) Date of publication of application: **25.09.2013**
(21) Application number: 11840951.5
(22) Date of filing: 18.11.2011
(51) Int. Cl.: A61K 31/202, A61K 31/232, A61K 31/355, A61K 31/661, A61K 35/60, A61P 27/02, A61P 43/00

(54) **THERAPEUTIC OR PROPHYLACTIC AGENT FOR CORNEAL EPITHELIUM DISORDERS AND/OR CONJUNCTIVAL EPITHELIUM DISORDERS**

(30) Priority: 19.11.2010 JP 2010258565
(71) Applicant: Nippon Suisan Kaisha, Ltd., Chiyoda-ku Tokyo 100-8686 (JP); School Corporation, Azabu Veterinary Medicine Education Institute, Kanagawa 252-5201 (JP); Keio University, Tokyo 108-8345 (JP)
(72) Inventor: HARAUMA, Akiko, Tokyo 100-8686 (JP); KAWABATA, Fuminori, Tokyo 100-8686 (JP); MORIGUCHI, Toru, Sagamihara-shi Kanagawa 2525201 (JP); KAWAKITA, Tetsuya, Tokyo 160-8582 (JP); TSUBOTA, Kazuo, Tokyo 160-8582 (JP)
(74) Representative: Grünecker, Kinkeldey, Stockmair & Schwanhäusser
(86) International application number: PCT/JP2011/076643
(87) International publication number: WO 2012/067224

(57) **Abstract**

The present invention provides an effective and safe prophylactic and therapeutic pharmaceutical agent and supplement for corneal epithelium disorders and/or conjunctival epithelial disorders. This is a therapeutic and/or prophylactic agent for corneal epithelium disorders and/or conjunctival epithelial disorders comprising eicosapentaenoic acid and/or docosahexaenoic acid, a glycerin ester or phospholipid comprising these fatty acids as constituent fatty acids, or a lower alcohol ester of these fatty acids as active ingredients. These preferably comprise EPA and/or DHA in the form of a refined fish oil or refined krill oil. A total of at least approximately 50 to 5,000 mg of EPA and/or DHA or the esters thereof are taken daily, thereby easing corneal epithelium disorders and/or conjunctival epithelial disorders. In addition, the amount of tears may be recovered.

## Description

### TECHNICAL FIELD

The present invention relates to the treatment and prevention of eye diseases such as corneal epithelium disorders and/or conjunctival epithelial disorders caused by irritation due to dry eyes, contact lenses, and eyelashes inverted towards the eyelids, conjunctival lithiasis, viral conjunctivitis, and allergic conjunctivitis.

### BACKGROUND ART

Along with the spread of personal computers, cell phones, and the like, the number of people suffering from eyestrain or feeling some kind of discomfort in their eyes is on the rise. Particularly, regarding contact lens users, complaints of discomfort such as dryness of the eyes, and gritty sensations caused by staring at the screen are increasing. Dryness of the eyes, that is, dry eye is attracting attention as the cause of said symptoms.
Dry eye is a disease in which a disorder is generated on the eye surface due to a decrease or qualitative change in tears, thereby causing the eyes to become dry and prone to damage due to declined amount of tears, and making the use of contact lenses difficult.
According to the diagnostic criteria for dry eye by the Dry Eye Society in 2006, dry eye is diagnosed when all three of: subjective symptoms (evaluation of pain and dryness by a VAS test); abnormal tear fluid (evaluation from tear film breakup time or Schirmer's test); and corneal as well as conjunctival disorders (evaluation by fluorescein or rose bengal staining) are confirmed, with dry eye suspected when two of these are confirmed.

Saline eye drops and Viscous eye drops such as chondroitin sulfate are used as symptomatic treatments against dry eye. Moreover, one treatment involves closing the tear outlet in order to retain tears on the eye surface for as long as possible.
Fine scratches generated on the corneal epithelium and/or conjunctival epithelium due to irritation due to contact lenses and eyelashes inverted towards the eyelids, conjunctival lithiasis, viral conjunctivitis, allergic conjunctivitis, and the like are also possible in addition to dry eye.

Nutritional supplements for dry eye involving the concomitant use of GLArich flaxseed oil, eicosapentaenoic acid (hereinafter, referred to as EPA) and/or docosahexaenoic acid (hereinafter, referred to as DHA) are described in Patent Document 1. The effect of essential fatty acids with respect to dry eye is described in Non-patent Document 1.

### BACKGROUND ART DOCUMENTS

### Patent Documents

Patent Document 1: Japanese Unexamined Patent Application Publication (Translation of PCT Application) No. 2005-535733

### Non-patent Documents

Non-patent Document 1: "Essential Fatty Acids in the Treatment of Dry Eye," The Ocular Surface, 8 (1) p 18-28, 2010.

### SUMMARY OF THE INVENTION

### Problems to be Solved by the Invention

Even when not serious, dry eye impairs the comfort of daily life. An object of the present invention is to investigate the cause of dry eye and provide a safe therapeutic strategy without the worry of side effects that are not symptomatic treatments.

### Means for Solving the Problems

In advancing studies on n-3 fatty acid-deficient mice, the inventors of the present invention found that the amount of tears in n-3 fatty acid-deficient mice declined, hypothesizing that n-3 fatty acid was involved in the amount of tears. Furthermore, they found that said amount of tears may be brought back to normal levels by administrating refined fish oil with high EPA and DHA content, which are n-3 fatty acids, to the n-3 fatty acid-deficient mice, thereby completing the present invention.

The present invention is summarized by the therapeutic and/or prophylactic agent for corneal epithelium disorders and/or conjunctival epithelial disorders of (1) to (7) and the tear quantity restorative agent of (8) to (13).
(1) A therapeutic and/or prophylactic agent for corneal epithelium disorders and/or conjunctival epithelial disorders, comprising EPA and/or DHA or esters thereof as an active ingredient.
(2) The therapeutic and/or prophylactic agent according to (1), wherein said corneal epithelium disorders and/or conjunctival epithelial disorders are disorders caused by any from the group consisting of: irritation due to dry eyes, contact lenses, and eyelashes inverted towards the eyelids, conjunctival lithiasis, viral conjunctivitis, and allergic conjunctivitis.
(3) The therapeutic and/or prophylactic agent according to (1) or (2), wherein the esters of EPA and/or DHA are comprised in the form of a glycerin ester, lower alcohol ester, phosphate ester or refined fish oil.
(4) The therapeutic and/or prophylactic agent according to any one of (1) to (3), for the purpose of ingesting a total of at least approximately 50 to 5,000 mg of eicosapentaenoic acid and/or docosahexaenoic acid or esters thereof daily.
(5) The therapeutic and/or prophylactic agent according to any one of (1) to (3), for the purpose of ingesting a total of at least approximately 300 to 3,000 mg of EPA and/or DHA or esters thereof daily.
(6) The therapeutic and/or prophylactic agent according to any one of (1) to (3), for the purpose of ingesting a total of at least approximately 1,000 to 2,000 mg of EPA and/or DHA or esters thereof daily.
(7) The therapeutic and/or prophylactic agent according to any one of (1) to (6), further comprising tocopherol as an antioxidant.

(8) A tear quantity restorative agent comprising EPA and/or DHA or ethers thereof as an active ingredient.
(9) The tear quantity restorative agent according to (8), wherein the esters of EPA and/or DHA are comprised in the form of a glycerin ester, lower alcohol ester, phosphate ester, or refined fish oil.
(10) The tear quantity restorative agent according to (8) or (9), for the purpose of ingesting a total of at least approximately 50 to 5,000 mg of eicosapentaenoic acid and/or docosahexaenoic acid or esters thereof daily.
(11) The tear quantity restorative agent according to (8) or (9), for the purpose of ingesting a total of at least approximately 300 to 3,000 mg of EPA and/or DHA or esters thereof daily.
(12) The tear quantity restorative agent according to (8) or (9), for the purpose of ingesting a total of at least approximately 900 to 2, 100 mg of EPA and/or DHA or esters thereof daily.
(13) The tear quantity restorative agent according to any one of (8) to (12), further comprising tocopherol as an antioxidant.

### Advantage of the Invention

Corneal epithelium disorders and/or conjunctival epithelial disorders caused by irritation due to dry eyes, contact lenses, and eyelashes inverted towards the eyelids, conjunctival lithiasis, viral conjunctivitis, allergic conjunctivitis, and the like may be treated or prevented by administrating EPA and DHA, which are n-3 fatty acids. EPA and DHA are capable of increasing the amount of tears, thereby easing said symptoms. Thus, a pharmaceutical agent, supplement, and the like capable of improving symptoms such as dry eye in the form of refined fish oil with a long dietary history and no worry of side effects may be provided.

### BRIEF DESCRIPTION OF THE DRAWINGS

FIG. 1 is a drawing showing the results from evaluating the subjective evaluation of eye pain using a VAS test. In the drawing, *P<0.05, #P<0.1 indicates the significant difference according to a Mann-Whitney U test.
FIG. 2 is a drawing showing the results from evaluating eye dryness using a VAS test.
FIG. 3 is a drawing showing the results from measuring the tear film breakup time. n=24 or 30. In the drawing, *P<0.05, #P<0.1 indicates the significant difference according to a Mann-Whitney U test.
FIG. 4 is a drawing showing the results from evaluating scratches on the eye using fluorescein staining. n=24 or 30.
FIG. 5 is a drawing showing the variation of FIG. 4.
FIG. 6 is a drawing showing the results from evaluating scratches on the eye using rose bengal staining. n=24 or 30.
FIG. 7 is a drawing showing the variation of FIG. 6. In the drawing, *P<0.05 indicates the significant difference according to a Mann-Whitney U test. In the drawing, **P<0.05 indicates the significant difference according to two-way ANOVA.
FIG. 8 is a drawing showing the results from evaluating the amount of tears using a Schirmer's test. n=24 or 30.

### MODE FOR CARRYING OUT THE INVENTION

The present invention is a prophylactic or therapeutic agent for corneal epithelium disorders and/or conjunctival epithelial disorders, comprising EPA and/or DHA or esters thereof as an active ingredient. Although several reports exist in which n-6 type fatty acids such as flaxseed oil are used as treatments for dry eye, it is unknown how the single ingestion of n-3 fatty acids (especially EPA and DHA) affect corneal epithelium disorders and/or conjunctival epithelial disorders.
The inventors of the present invention found that a lack of n-3 fatty acid evidently decreases the amount of tears and confirmed that said amount of tears is recovered by the ingestion of refined fish oil comprising EPA and DHA.

The EPA and/or DHA used in the present invention is preferably an ester with a glycerol, that is, triglyceride, diglyceride, monoglyceride, or phospholipid, that is, phosphatidylcholine, phosphatidylserine, phosphatidyl ethanolamine, phosphatidylinositol, phosphatidylglycerol, cardiolipin, phosphatidic acid (all comprising lysozymes body), or esters with lower alcohol (carbon number: 1 to 5), that is, methyl ester, ethyl ester, and the like. Those with n-3 fatty acids such as EPA, and DHA as the main constituent, which do not substantially contain n-6 type fatty acids, are preferable. Specifically, the total amount of n-6 type fatty acid is preferable 10% by weight or less and more preferably 5% by weight or less.
Specifically, refined fish oil, concentrated oil (oil with the EPA and DHA of refined fish oil concentrated by enzyme reactions or chemical reactions), and the like may be used as a triglyceride. Moreover, krill oil, and the like may be used as a phospholipid. Sources of n-3 polyunsaturated fatty acid comprising EPA and DHA include: fish or shellfish extract, animal extract, egg yolk extract, plant extract, fungi extract, and the like, preferably krill oil, fish oil, fish extract, squid extract, bonito ovary extract, extracts from genetically modified plants, extracts from labyrinthulea, and the like. Krill oil, squid extract, and bonito ovary extract are substances with high phospholipid content. By means of techniques involving concentrating, extracting, and/or refining, mixing, and the like that are generally known in technical fields, the phospholipid concentration and purity of said substances may be adjusted as required.
EPA and/or DHA are known to be effective for hyperlipidemia and allergy symptoms, with many pharmaceutical agents and various supplements already commercially available. A capsule form similar to these may be used for the purpose of the present invention. Moreover, it is possible to ingest these by adding them to various beverages and food products.
Specifically, gelatin capsules processed from oils and fat with antioxidants added to refined fish oil are often used.

The refined fish oil may be fish oil from any fish as long as said fish oil contains EPA and DHA. Examples of fish oil with high EPA content include sardine oil, cod liver oil, and the like, while examples of fish oil with high DHA content include tuna oil, bonito oil, and the like.
Concentrated EPA/DHA oil refers to fats or oils with increased EPA/DHA concentrations in the triglyceride of refined fish oil using lipase, and the like due to regioselectivity and fatty acid selectivity (refer to Japanese Examined Patent Application Publication No. H4-16519).

The therapeutic and/or prophylactic agent of the present invention is orally administered to patients with dry eye symptoms with a total quantity of EPA and/or DHA or ester thereof of approximately 50 to 5,000 mg, preferably 300 to 3,000 mg, more preferably approximately 900 to 2,100 mg, and most preferably 1,100 to 1,900 mg daily either at once or by dividing the dosage. Effects are observed upon continuous administration for at least 4 weeks.
EPA and/or DHA have a long history of usage with other methods, so there are believed to be no problems with regards to safety.

Anthocyanidin, lutein, eye bright, acer maximowiczianum, vitamins, and the like, which have been determined as favorable for recovery from eye fatigue, may be added to the therapeutic and/or prophylactic agent for corneal epithelium disorders and/or conjunctival epithelial disorders or the tear quantity restorative agent of the present invention.

The present invention will now be explained in greater detail through the use of working examples, but is in no way limited to these working examples.

### Working Example 1

### Creating an n-3 fatty acid-deficient animal

Experimental animal feeds generally comprise n-3 fatty acid with an appropriate DHA content; therefore, in order to create an n-3 fatty acid-deficient animal, a special feed that does not contain n-3 fatty acid must be used. Furthermore, normally raised animals receive sufficient DHA ingestion from the placenta and the dam's breast milk from prior to birth to the weaning period, so it is no simple matter to create a target n-3 fatty acid-deficient animal over a single generation. Taking these facts into consideration, an n-3 fatty acid-deficient feed (n-3 Def, linolenic acid, 14.4%; α-linolenic acid, 0.1 %) comprising 7% fat and oil was prepared with AIN93G (standard refined feed composition for nutrition research of mice and rats presented by the American Institute of Nutrition) as the basal feed. The n-3 fatty acid (n-3 Adq, linolenic acid, 14.3%; α-linolenic acid, 2.6%) containing α-linolenic acid (18:3, n-3) was used as the normal feed. These feeds were fed to female mice immediately following weaning (3 weeks old), which were then raised; subsequently, the mice were crossbred after maturing to obtain second generation mice, and said second generation male mice, aged 11 months old, were used for the experiment. Moreover, in order to avoid any effects from the dam upon conducting the experiment, the test was set such that the samples in each group all had different mothers.

### Preparing the test drug

Fish oil (refined fish oil EPA-28 (containing 28% or more of EPA and 12% or more of DHA) manufactured by Nippon Suisan Kaisha, Ltd.) was used as the test drug, and the dosage was adjusted such that the DHA content became 5 mg/head and the EPA content became 11 mg/head. Palm oil was used in the control group, which was prepared such that it had the equivalent fatty acid content of the fish oil group. The principal fatty acid composition of fish oil is shown in Table 1 (the n-6 type fatty acid content of 18:2 was 1.1% and 20:4 was 1.3%).

**Table 1**

| Fatty acid | (%) |
|---|---|
| 14:0 | 4.9 |
| 16:0 | 6.2 |
| 16:1 | 8.7 |
| 16:4 | 4.8 |
| 18:1 | 8.3 |
| 18:4 | 5.1 |
| 20:5 (EPA) | 29.1 |
| 22:6 (DHA) | 15.0 |

### Operation procedure

A phenol red thread (Zone-Quick, SHOWA YAKUHIN KAKO CO., LTD.) for checking the amount of tear fluid was used to measure the amount of tears (the length at which the thread indicates a red color) secreted within 30 seconds in n-3 Def and n-3 Adq mice. These mice were divided into two groups and each group was orally administered fish oil or palm oil-mixed feed for 7 days; subsequently, the amount of tear fluid was measured again on day 8, and on day 9, the retina related to the vision and the lachrymal gland and the meibomian gland related to the secretion of tear fluids were sampled and the fatty acid composition was measured.

### Experimental results and discussion

Tear fluids are configured from a lipid layer secreted from the meibomian gland, an aqueous layer secreted from the lachrymal gland, and a mucus layer secreted from the conjunctiva in order, with the layers contacting the exterior being first. Dry eye is also referred to as corneal xerosis and is an eye disease in which scratches and disorders are generated due to the surface of the eyeballs drying, accompanied by eye discomfort and impaired visual function; wherein, a tear deficient dry eye in which the tear fluid of the aqueous layer secreted from the lachrymal gland declines, and an evaporative dry eye caused due to insufficient lipid layer secreted from the meibomian gland are known.

A clear decline was exhibited in the amount of tear fluid in elderly n-3 fatty acid-deficient mice, suggesting the exhibition of dry eye symptoms (Table 2). The fish oil group in which fish oil was administered to said n-3 fatty acid-deficient mice for 7 days was observed to exhibit a significant increase in the amount of tears compared to the control group (recovered amount of tears) (Table 3). Moreover, because said amount of tears was almost the same as that of normal mice, it was believed that the amount of tears had almost completely recovered. From the results of the fatty acid composition of the EPA and DHA of organs involved with the amount of tears, an increase was observed in both the lachrymal gland and meibomian gland due to fish oil administration (Table 4).

From the results above, it became clarified that n-3 fatty acid-deficient animals exhibit evident dry eye symptoms, and that said symptoms recover due to the ingestion of fish oil. Moreover, from the reaction results of the fatty acid composition following fish oil administration, it was believed that the dry eye symptoms of n-3 fatty acid-deficient animals were caused by the decreased amount of tear fluid secretion due to decreased functioning of both or either of the lachrymal gland and meibomian gland.

**Table 2**

| | Number of animals | Amount of tear fluid (mm) |
|---|---|---|
| | | (Mean±SEM) |
| n-3 Adq mice (normal mice) | 18 | 9.64±0.79 |
| n-3 Def mice | 17 | 5.91±0.59** |

### (**:P<0.01, t-test)

**Table 3**

| | Normal mice | | n-3 fatty acid-deficient mice | |
|---|---|---|---|---|
| | Control group (n=8) | Fish oil group (n=10) | Control group (n=9) | Fish oil group (n=8) |
| Amount of tears (mm) | 11.97±1.69 | 11.71±1.98 | 7.07±0.89 | 11.75±2.11* |

### (*:P<0.05, vs Control group, t-test)

**Table 4**

| Fatty acid composition of the main organization (percentage with respect to the total fatty acid, %) | | | | | |
|---|---|---|---|---|---|
| | | Normal mice | | n-3 fatty acid-deficient mice | |
| | | Control group (n=8) | Fish oil group (n=10) | Control group (n=9) | Fish oil group (n=8) |
| Retina | EPA | 0.29±0.02 | 0.44±0.03** | 0.02±0.01 | 0.02±0.02** |
| | DHA | 25.25±0.55 | 25.87±0.70 | 15.79±1.03 | 20.50+0.75** |
| Lachrymal gland | EPA | 0.88±0.09 | 1.41±0.12** | 0.12±0.01 | 0.65±0.05** |
| | DHA | 10.07±0.61 | 10.42±0.57 | 2.05±0.19 | 3.25±0.28** |
| Meibomian gland | EPA | 0.12±0.01 | 0.19±0.01** | 0.08±0.005 | 0.17±0.01** |
| | DHA | 1.60±0.09 | 2.10±0.06** | 0.25±0.01 | 1.28±0.13** |

### (**:P<0.01, vs Control group, t-test)

### Working Example 2

Using the same fish oil (refined fish oil EPA-28 (containing 28% or more of EPA and 12% or more of DHA) manufactured by Nippon Suisan Kaisha, Ltd.) as in Working Example 1, the effect on human corneal epithelium disorders was confirmed.

### Abstract of the test protocol

The efficacy of EPA and DHA was evaluated using a double-blind test with respect to the 27 cases of subjects diagnosed with dry eye from the diagnostic criteria for dry eyes (2006, Dry Eye Society). Active or placebo supplements were taken for 12 weeks while continuing normal dry eye treatment using eye drops, and the like. Capsules of refined fish oil (15 capsules) were administered to the active group for the purpose of ingesting 1,245 mg of EPA and 540 mg of DHA daily. Capsules of medium chain triglyceride (MCT) (15 capsules) were administered to the placebo group. The efficacy was measured a total of 5 times: prior to administration; week 4, week 8, and week 12 following administration; and week 4 of the follow-up period subsequent to administration.

The following 6 items were evaluated:
- Subjective evaluation of eye pain by the VAS test: the degree of pain was visually evaluated using a visual analog scale.
- Subjective evaluation of dryness of the eyes by the VAS test: the degree of dryness was visually evaluated using a visual analog scale.
- Tear film breakup time (BUT, tear film breakup time): one of the diagnostic criteria for dry eye. In this test, subjects are asked to cease blinking after fluorescein is instilled, after which the time until the tear film of the corneal surface ruptures is measured. The retention of tear fluid is measured. 10 seconds or more is evaluated as being normal, with 5 seconds or less evaluated as being suspicious of dry eye.
- Evaluation of scratches on the eyes using fluorescein staining: a yellow staining liquid called fluorescein is instilled to stain scratches on the eye surface in order to evaluate the seriousness of dry eye. The fluorescein also stains the conjunctiva, but it mainly allows the evaluation of scratches on the cornea.
- Evaluation of scratches on the eyes using rose bengal staining: a red staining liquid called rose bengal is instilled to stain scratches on the eye surface in order to evaluate the seriousness of dry eye. Rose bengal mainly allows the evaluation of the conjunctiva with an impaired mucus layer.
- Evaluation of the amount of tears using Schirmer's test: Schirmer's test is a test for measuring the amount of tear fluid secretion, involving sandwiching a long and narrow filter paper on the lower eyelids for 5 minutes and measuring the length of wetness from tears. 5 mm or less is evaluated as an abnormal value.

### Results

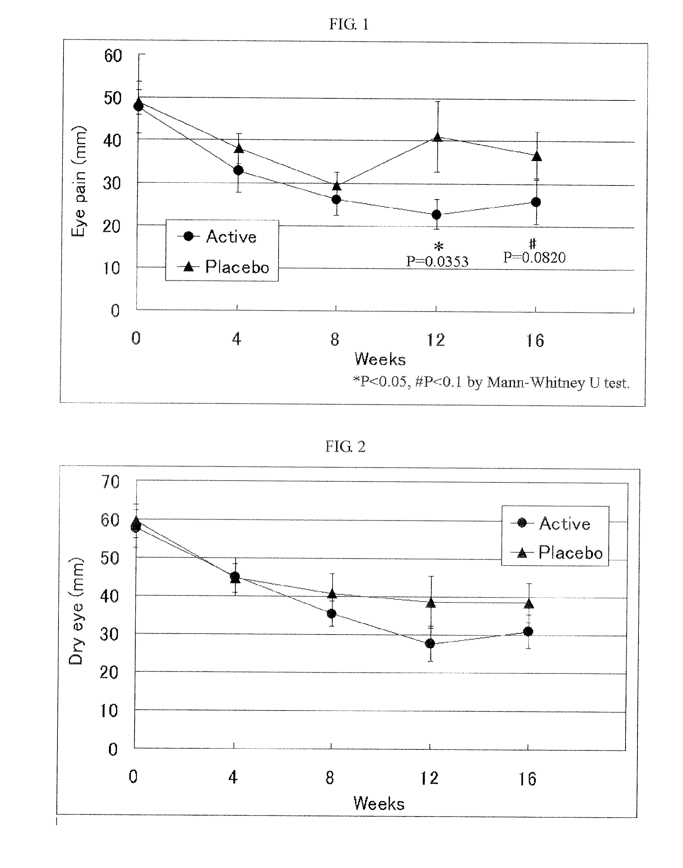

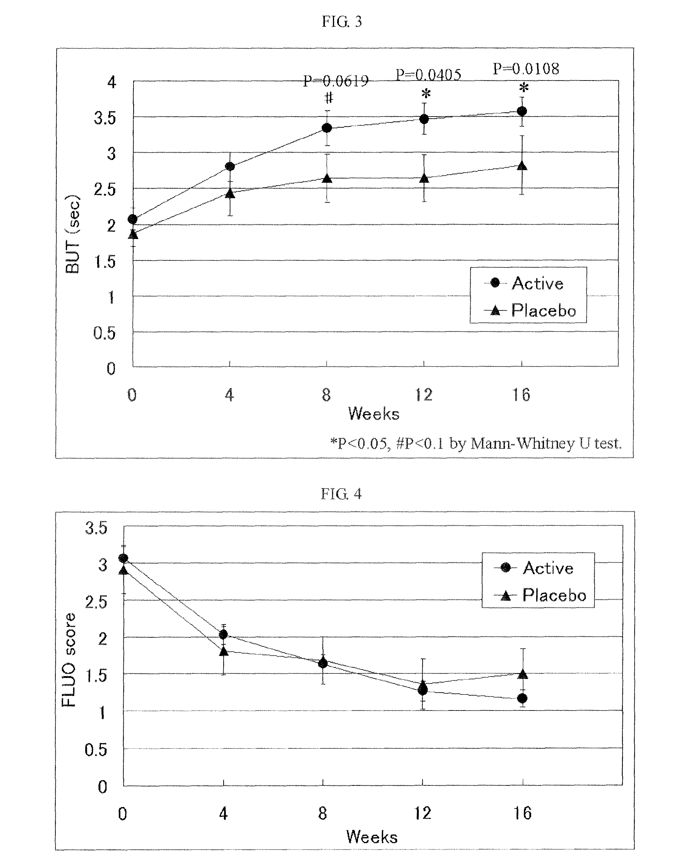

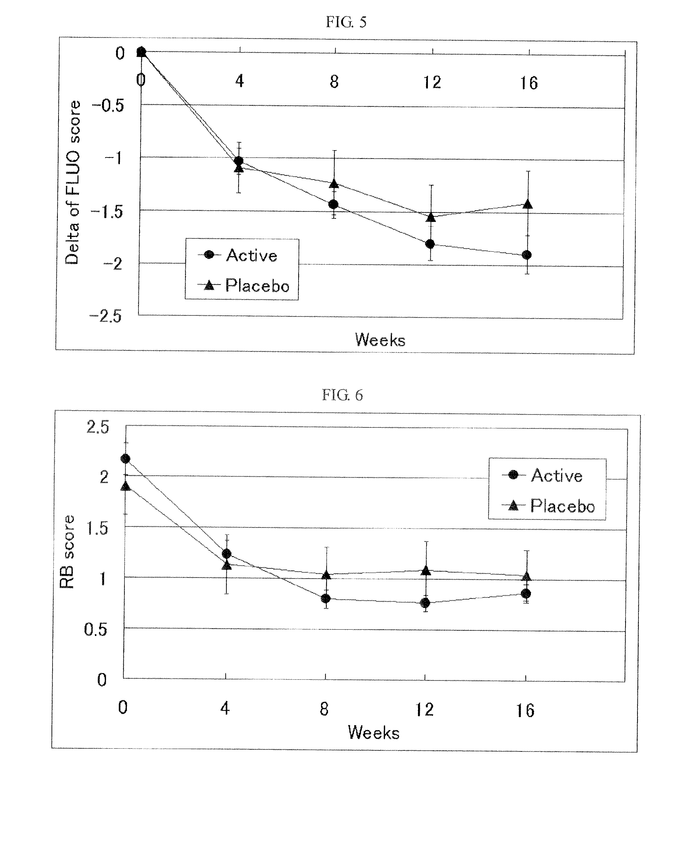

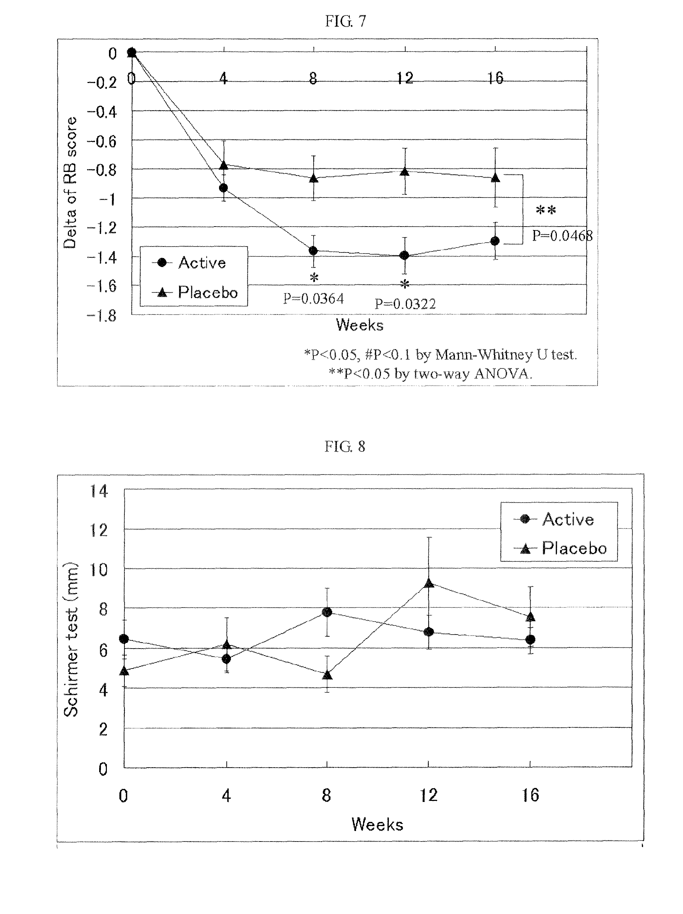

The number of subjects was 12 in the active group and 15 in the placebo group.
The results of each evaluation item are shown in FIGS. 1 to 8.
It was found that there was an effect with a statistical difference compared to the placebo group regarding the evaluation of eye pain by the VAS test, tear film breakup time, and the rose bengal staining test. In other evaluations as well, a tendency was observed in which the results were more favorable in the active group compared to the placebo group.

### Working Example 3

The effect was compared between the active group that took 162 mg of EPA, 784 mg of DHA, 59 mg of anthocyanidin, and 17 mg of lutein daily in the form of a capsule and the placebo group that took capsules comprising the medium chain triglyceride using a double-blind randomized comparative test targeting patients having eye strain symptoms.

### Result

The number of subjects was 11 in the active group and 9 in the placebo group. Upon a subjective symptom evaluation at week 4 following the commencement of administration, a statistically significant improvement in terms of dryness from among the eye symptoms was observed in the active group compared to the placebo group. Moreover, from among the eye symptoms, significant improvement in the feeling of fatigue, flickering, bloodshot eyes, and dimness of sight was observed in the active group at week 4 following the commencement of administration compared to prior to administration.

### INDUSTRIAL APPLICABILITY

The present invention can provide the pharmaceutical agent and supplement for easing eye disorders, especially corneal epithelium disorders and conjunctival epithelium disorders due to various causes such as irritation due to dry eye, use of contact lenses, and eyelashes inverted towards the eyelids, conjunctival lithiasis, viral conjunctivitis, and allergic conjunctivitis, and improving the feeling of discomfort, and the like.

## Claims

1. A therapeutic and/or prophylactic agent for corneal epithelium disorders and/or conjunctival epithelial disorders, comprising: eicosapentaenoic acid and/or docosahexaenoic acid, a glycerin ester or a phospholipid containing said fatty acids as constituent fatty acids, or a lower alcohol ester of said fatty acids as an active ingredient.

2. The therapeutic and/or prophylactic agent according to claim 1, wherein said corneal epithelium disorders and/or conjunctival epithelial disorders are disorders caused by any from the group consisting of: irritation due to dry eyes, contact lenses, and eyelashes inverted towards the eyelids; conjunctival lithiasis; viral conjunctivitis; and allergic conjunctivitis.

3. The therapeutic and/or prophylactic agent according to claim 1 or 2, wherein the glycerin ester or the phospholipid containing the eicosapentaenoic acid and/or the docosahexaenoic acid as the constituent fatty acid is comprised in the form of a refined fish oil or a refined krill oil.

4. The therapeutic and/or prophylactic agent according to any one of claims 1 to 3, for the purpose of ingesting a total of approximately 50 to 5,000 mg of eicosapentaenoic acid and/or docosahexaenoic acid daily.

5. The therapeutic and/or prophylactic agent according to any one of claims 1 to 3, for the purpose of ingesting a total of approximately 300 to 3,000 mg of eicosapentaenoic acid and/or docosahexaenoic acid daily.

6. The therapeutic and/or prophylactic agent according to any one of claims 1 to 3, for the purpose of ingesting a total of approximately 900 to 2,100 mg of eicosapentaenoic acid and/or docosahexaenoic acid daily.

7. The therapeutic and/or prophylactic agent according to any one of claims 1 to 6, further comprising tocopherol as an antioxidant.

8. A tear quantity restorative agent, comprising: eicosapentaenoic acid and/or docosahexaenoic acid, a glycerin ester or a phospholipid containing said fatty acids as constituent fatty acids, or a lower alcohol ester of said fatty acids as an active ingredient.

9. The tear quantity restorative agent according to claim 8, wherein the glycerin ester or the phospholipid containing the eicosapentaenoic acid and/or the docosahexaenoic acid as the constituent fatty acid is comprised in the form of a refined fish oil or a refined krill oil.

10. The tear quantity restorative agent according to claim 8 or 9, for the purpose of ingesting a total of approximately 50 to 5,000 mg of eicosapentaenoic acid and/or docosahexaenoic acid daily.

11. The tear quantity restorative agent according to claim 8 or 9, for the purpose of ingesting a total of approximately 300 to 3,000 mg of eicosapentaenoic acid and/or docosahexaenoic acid daily.

12. The tear quantity restorative agent according to claim 8 or 9, for the purpose of ingesting a total of approximately 900 to 2,100 mg of eicosapentaenoic acid and/or docosahexaenoic acid daily.

13. The tear quantity restorative agent according to any one of claims 8 to 12, further comprising tocopherol as an antioxidant.
